# EUROPEAN PATENT SPECIFICATION

(11) **EP 3 701 981 B1**
(45) Date of publication and mention of the grant of the patent: **05.08.2026**
(21) Application number: 19206717.1
(22) Date of filing: 18.08.2009
(51) Int. Cl.: A61M 5/142, A61M 5/158, A61M 5/145

(54) **DRUG INFUSION SYSTEM WITH REUSABLE AND DISPOSABLE COMPONENTS**
WIRKSTOFFINFUSIONSSYSTEM MIT WIEDERVERWENDBAREN UND EINWEG-KOMPONENTEN
SYSTÈME DE PERFUSION DE MÉDICAMENT COMPORTANT DES COMPOSANTS RÉUTILISABLES ET JETABLES

(30) Priority: 18.08.2008 US 89749 P; 21.07.2009 US 227157 P
(43) Date of publication of application: 02.09.2020
(62) Divisional of application: 09808725.7
(73) Proprietor: Ethicon, Inc., Raritan, NJ 08869 (US)
(72) Inventor: MCKENZIE, John, San Carlos, California 94070-4850 (US); CROSS, Brett, Seattle, Washington 98119-2814 (US); MARSOT, Travis, Mountain View, California 94043 (US)
(74) Representative: Carpmaels & Ransford LLP

(56) References cited:
- EP-A2- 0 856 331
- WO-A1-2007/071255
- WO-A2-2008/024812
- US-A- 4 734 092
- US-A1- 2002 022 804
- US-A1- 2004 204 673
- US-A1- 2006 200 073
- US-B1- 6 277 095

## Description

### BACKGROUND OF THE INVENTION

The present invention relates to infusion devices and more particularly to such devices that enable liquid medicaments to be conveniently and safely self-administered by a patient. One liquid medicament that is often self-administered by a patient is insulin, and for ease of description, the administration of insulin is generally used herein for exemplary purposes although the invention should not be limited by that exemplary use.

Administration of insulin has traditionally been accomplished using a syringe. Recently, needle carrying pen-like devices have also been employed for this purpose. Both forms of insulin administration require the patients to stick themselves each time they inject insulin, often many times a day. Additionally, a new clean needle must be mounted on the device each time they are used, and disposed of after each use, creating the additional problem of having the "sharps" with them whenever the patient needs to administer insulin, and to safely dispose of them after each use. Thus, these traditional forms of insulin administration have been a rather pervasive intrusion in the lives and routines of the patients who have had to adopt and employ them.

More recently, insulin pumps attached by tubing to an infusion set mounted on the patient's skin have been developed as an alternative form of insulin administration. Such pumps may be controlled by a programmable remote electronic system employing short range radio communication between a control device and electronics that control the pump. While such devices may involve fewer needle sticks, they are expensive to manufacture. They are also complex to operate and cumbersome and awkward to wear. Further, the cost of such devices can be many times the daily expense of using a traditional injection means such as a syringe or an insulin pen.

Devices of the type mentioned above also require a significant amount of training to control and thus use the devices. Great care in programming the devices is required because the pumps generally carry sufficient insulin to last a few days. Improper programming or general operation of the pumps can result in delivery of an excessive amount of insulin which can be very dangerous and even fatal.

Many patients are also reluctant to wear a pump device because they can be socially awkward. The devices are generally quite noticeable and can be as large as a pager. Adding to their awkwardness is their attachment to the outside of the patients clothes and the need for a catheter like tubing set running from the device to an infusion set located on the patient's body. Besides being obvious and perhaps embarrassing, wearing such a device can also be a serious impediment to many activities such as swimming, bathing, athletic activities, and many activities such as sun bathing where portions of the patient's body are necessarily uncovered.

In view of the above, a more cost effective and simple device has been proposed whereby an injection system is discreetly attached directly to the skin of the patient. One example of such a device is described in detail in U.S. Application 12/147,283 filed June 26, 2008 and titled DISPOSABLE INFUSION DEVICE WITH REDUNDANT VALVED SAFETY Such a device may be attached to the patient under the patient's clothing to deliver insulin into the patient by the manual pumping of small doses of insulin out the distal end of a temporarily indwelling cannula that is made a part of the pump device. The device may be made quite small and, when worn under the clothes, entirely unnoticeable in most social situations. It may still carry sufficient insulin to last a patient several days. It can be colored to blend naturally with the patient's skin color so as not to be noticeable when the patient's skin is exposed. As a result, insulin for several days may be carried by the patient discreetly, and conveniently applied in small dosages after only a single needle stick. For another description of devices of this type, reference may also be had to co-pending application Serial Number 11/906,130, filed on September 28, 2007 for DISPOSABLE INFUSION DEVICE WITH DUAL VALVE SYSTEM.

Although relatively discrete, the patient may have a reason to remove the system entirely. Likewise, if the drug delivery system is accidentally dislodged from the patient, it would be advantageous to be able to salvage the medicament and pump, and to replace only the minimum amount of the system. Where the pump, insulin supply and cannula are integral and non-separable units, removing just the pump or just the insulin, or adding a different liquid medicament is not generally feasible. Sometimes it would be advantageous to be able to remove the pump unit, the insulin reservoir, or the entire device, and to reassemble and use parts of the drug delivery system. Additionally, since the portion of system that contains the cannula needs to be removed and reinstalled every three days pursuant to current medical and regulatory practice, it would be advantageous to be able to remove the other portions of the drug delivery system from the portion with the cannula, and reattach them to a new cannula containing portion, thus avoiding replacing them with every use.

Further, it would be advantageous if the device was configured to utilize commercially available reservoirs or cartridges. For example, glass cartridges are presently used for insulin injection pens, which are readily available to the patient with a prescription. It would be beneficial to some patients to combine the availability of these cartridges with a discreet device that removes the attendant problems of a syringe-pen. Such a device would also decrease the attendant manufacturing costs of a device that utilizes proprietary reservoirs. More importantly, it would mitigate the inconvenience to the patient of filling or refilling a reservoir and the attendant problems associated with the patient performing that task.

Therefore there is a need for an invention that makes it possible to have a small, simple and discreet drug delivery system and yet be able to remove various components of the drug delivery system from each other, and to reattach them to each other without the need to discard the entire system.

For further background, WO 2008/024812A describes a delivery device that includes a durable housing portion and a separable disposable portion that selectively engage and disengage from each other. The disposable housing portion secures to the patient-user and may be disposed of after it has been in use for a prescribed period. Components that normally come into contact with a patient-user or with infusion media are supported by the disposable housing portion for disposal after the prescribed use, while the durable housing portion supports other components, such as electronics for controlling delivery of infusion media from the reservoir and a drive device and drive linkage.

EP 0856331A describes a medical adapter having both a needleless valve and a sharpened cannula, which is used to connect or adapt pierceable septa containers or other devices having different sizes to needleless connection. The adapter includes a needleless site at one end and a sharpened cannula at the other end protected by spring arms. These arms include claws at their distal ends to grasp the neck of the pierceable septum device to which the sharpened cannula is to be inserted. The claws include sharpened points for gripping the device. The arms are located on either side of the adapter body and are connected to the body through springs. Handles are also included on the arms for use by the operator to separate the arms against the spring forces during engagement of the adapter with the septum.

US2006200073A1 discloses a medical device is provided, comprising a mounting surface adapted for application to the skin of a subject, a first portion having a first end adapted to penetrate the skin of the subject, and a second portion in fluid communication with the first portion and having a second end. The device further comprises enclosure means being transformable from an initial configuration encapsulating the first and second portions in an initial
aseptic state, to a second configuration in which the ends of the first and second portions are allowed to communicate with the exterior through the enclosure means, wherein the enclosure means does not enclose the mounting surface.

WO2007071255A1 discloses a device for an intermittent or continuous administration of a therapeutical substance, such as insulin, comprising an injection part and a fluid delivery part. The fluid delivery part comprises a reservoir, a pump and a house, and the injection part comprising a base plate, a cannula part comprising a cannula extending past the proximal side of the base plate and means for fixation of the base plate to the skin of the user.

### SUMMARY OF THE INVENTION

The invention is defined by the appended independent claim. Embodiments are defined by the dependent claims.

### BRIEF DESCRIPTION OF THE DRAWINGS

The features of the present invention are set forth with particularity in the appended claims. The invention, together with further
features and advantages thereof, may best be understood by making reference to the following description taken in conjunction with the accompanying drawings, in the several figures of which like reference numerals identify identical elements, and wherein:
**FIG. 1** is a top perspective view of a drug infusion system;
**FIG. 2** is a bottom plan view of the drug infusion system of **FIG. 1****;**
**FIG. 2B** is a simplified side view of an alternative embodiment of the device shown in **FIGS. 1 and 2****;**
**FIG. 2C** is a side view of the embodiment of **FIG.** 2B during cannula insertion;
**FIG. 2D** is a side view of the embodiment of **FIG.** 2B after cannula insertion;
**FIG. 3** is a top perspective view of the base portion of the system of **FIG. 1****;**
**FIG. 4** is a top perspective view of the base portion of the system of **FIG. 1** with a cannula set aligned therewith for deployment;
**FIG. 5** is a partial sectional side view, to an enlarged scale, illustrating details of the cannula set and base portion prior to cannula set deployment;
**FIG. 6** is a partial sectional side view, to an enlarged scale, illustrating details of the cannula set and base portion after cannula set deployment;
**FIG. 7** is a bottom perspective view of the reusable portion of the system of **FIG. 1****;**
**FIG. 8** is a top perspective view of the reusable portion being mated with the base portion of the system of **FIG. 1****;**
**FIG. 9** is a side view in section, to an enlarged scale, of the antiseptic coupling between the base portion and the reusable portion prior to their engagement;
**FIG. 10** is a side view in section, to an enlarged scale, of the antiseptic coupling between the base portion and the reusable portion during the process of their engagement;
**FIG. 11** is a side view in section, to an enlarged scale, of the antiseptic coupling between the base portion and the reusable portion after their engagement;
**FIG. 12** is a side view in section, to an enlarged scale, of the antiseptic coupling between the base portion and the reusable portion of another drug infusion system;
**FIG. 13** is a perspective view of another drug infusion system having detachable
   pump component and reservoir component;
**FIG. 14** is a top plan view of the system of **FIG. 13** showing reservoir and pump components thereof aligned for engagement;
**FIG. 15** is a top plan view of the system of **FIG. 13** showing the reservoir and pump components thereof entering engagement; and
**FIG. 16** is a top plan view of the system of **FIG. 13** showing the reservoir and pump components thereof after engagement.
**FIG 17** is a side view in section of the attachment mechanism between the reservoir portion and pump portion of one embodiment;
**FIG 18** is a side sectional view to an expanded scale of the connection mechanism between the reservoir portion and the pump portion of one embodiment of the invention;
**FIG. 19A** is a top view, in perspective, of an embodiment using a commercially available cartridge;
**FIG. 19B** is a bottom view of the embodiment of **FIG.19A****;**
**FIG. 19C** is a partial side view, in section, of the embodiment of **FIG. 19A****;**
**FIG. 20A** is a top plan view of another embodiment using a commercially available cartridge;
**FIG. 20B** is a side view, in section, of the embodiment of FIG. **20A****;**
**FIG. 20C** is a side view of the embodiment of **FIG.** 20A;
**FIG. 20D** is a top view of the embodiment of **FIG. 20A****;**
**FIG. 21A** is a top view of another embodiment using a commercially available cartridge;
**FIG. 21B** is an end view of the embodiment of **FIG.** 21A;
**FIG. 22** is an exploded view of the components of a further infusion assembly;
**FIG. 23** is a perspective view of the assembled components of the assembly of FIG. 22;
**FIG. 24** is a bottom view, in perspective, of the assembled components of the assembly of FIG. 22 prior to deployment on a user;
**FIG. 25** is a perspective view, with portions cut away, illustrating the releasable lock of the cartridge reservoir within the pump unit;
**FIG. 26** is a top plan view, with portions removed, illustrating the cartridge reservoir being loaded into the pump unit and just prior to being releasably locked therein;
**FIG. 27** is a top plan view, with portions removed, illustrating the cartridge reservoir after being loaded into the pump unit and being releasably locked therein;
**FIG. 28** is an end view, in perspective, of the base unit of the assembly of **FIG. 22****;**
**FIG. 29** is a top plan view of the assembled assembly of **FIG. 22****,** with portions removed, illustrating a first condition of a compression spring contacting a cartridge reservoir;
**FIG. 30** is a side plan view, with portions removed, of the assembled assembly of **FIG. 29****;**
**FIG. 31** is a top plan view of the assembled assembly of **FIG. 29****,** with portions removed, illustrating a second condition of the compression spring;
**FIG. 32** is a side plan view, with portions removed, of the assembled assembly of **FIG. 31****;**
**FIG. 33A** is a top plan view of the assembled assembly of **FIG. 29****,** with portions removed, illustrating a nearly empty condition of the cartridge reservoir and the compression spring;
**FIG. 33B** is a side plan view, with portions removed, of the assembled assembly of **FIG. 33A****;**
**FIG. 34A** is a top plan view of the assembled assembly of **FIG. 22****,** with portions removed, of a further embodiment of a compression spring that assists throughout fluid delivery from a cartridge reservoir;
**FIG. 34B** is a side plan view, with portions removed, of the assembled assembly of **FIG. 34A****;**
**FIG. 35** is an exploded view, in perspective, of the pump unit and cartridge reservoir of the assembly of **FIG. 22** just prior to the loading of the cartridge reservoir into the pump unit;
**FIG. 36** is a perspective view of the pump unit and cartridge reservoir during the loading of the cartridge reservoir into the pump unit;
**FIG. 37** is a perspective view of the pump unit and cartridge reservoir after the loading of the cartridge reservoir into the pump unit;
**FIG. 38** is a perspective view of the pump unit and base of the assembly of **FIG. 22** during the placement of the pump unit onto the base;
**FIG. 39** is a bottom plan view of the assembled assembly of **FIG. 22** shown during a priming process;
**FIG. 40A** is an exploded side plan view of the infusion device and an inserter for deploying the device in accordance with further aspects;
**FIG 40B** is an exploded view, in perspective, of the infusion device and inserter of **FIG. 40A****;**
**FIG. 41A** is a perspective view of the infusion device and inserter of FIG. **40A** after the infusion device has been loaded into the inserter;
**FIG. 41B** is a perspective view of the infusion device and inserter of FIG. **40A** after the infusion device has been loaded into the inserter and after a protective cannula cover and one adhesive cover have been removed from the device;
**FIG. 42** is a side plan view of the inserter, with the infusion device therein, against a patient's skin ready to deploy the device on the patient;
**FIG. 43** is a side plan view of the deployed device having an insertion needle removed therefrom;
**FIG. 44** is a perspective view of the insertion needle being safely stored in a cannula protector of the assembly of **FIG. 22** for sharps disposal;
**FIG. 45** is a perspective view of the deployed device on a patient's skin;
**FIG. 46** is a perspective viewing which may be interpreted as showing either a pump unit being removed from a deployed base or a replacement pump unit being placed on a deployed base; and
**Fig. 47** is a perspective view which may be interpreted as showing either a replacement pump being placed on a replacement base or a partially used pump unit being placed on a replacement base prior to deployment of the replacement base.

### DETAILED DESCRIPTION OF THE INVENTION

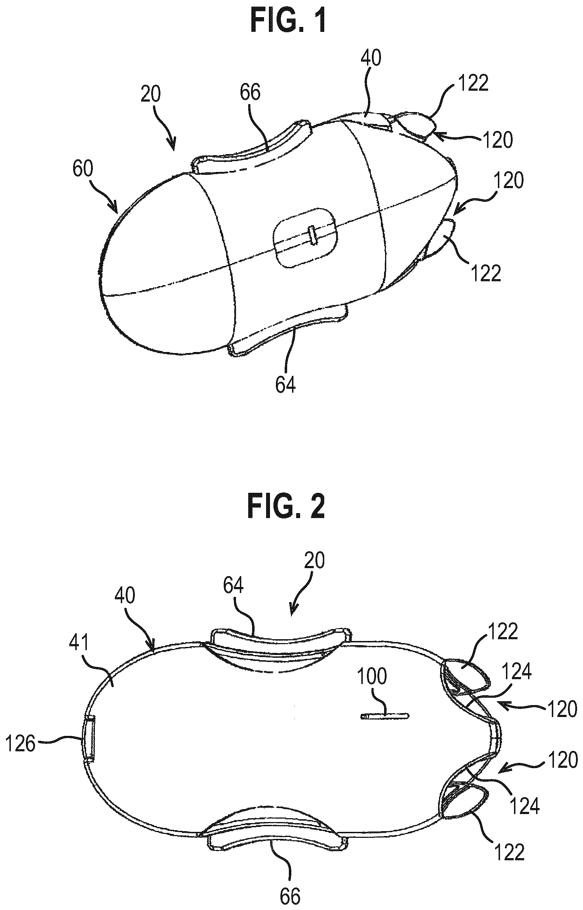

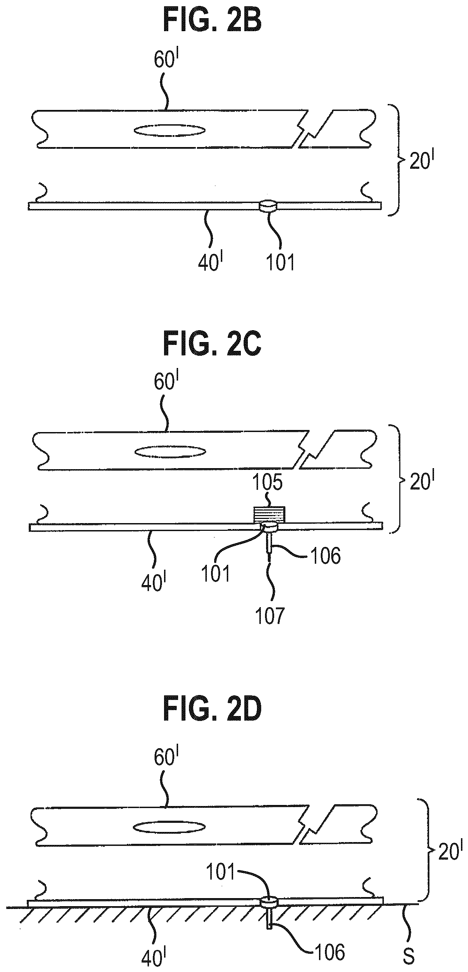

Referring now to **FIGS. 1 and 2****,** they show a drug infusion system **20.** The system **20** generally includes a lower base portion **40** and a reusable drug dispenser portion **60.** As will be seen subsequently, the reusable portion is arranged to be releasably attached to the base portion **40.** In **FIGS. 1** and
2, the base portion **40** and reusable portion **60** are fully engaged or joined together.

The base portion **40** includes a base surface **41** which preferably is coated with an adhesive for attaching the base portion **40** to the skin of a wearer in need of the drug, such as insulin, to be delivered by the system **20.** To that end, the base **40,** in a manner to be fully described herein after, is arranged to receive a cannula **100** which, when deployed, extends from the base surface **41** to beneath the skin of the wearer for subcutaneous delivery of the drug. The reusable dispenser portion includes a reservoir (not shown) for containing the drug and a pump (not shown) that, when actuated, pumps the drug from the reservoir to the cannula for delivery. As will be seen subsequently, when the base **40** and reusable portion **60** are joined together, a coupling arrangement provides an antiseptic connection there between. Also, the cannula **100** is a part of a cannula set which may be replaced in the base **40** when the reusable portion **60** is removed.

To actuate the pump, the reusable portion **60** includes a pair of actuator buttons **64** and **66.** Preferably, the actuator buttons are arranged with the pump and other operative internal components of the reusable portion **60** so that concurrent depression of the actuator buttons **64** and **66** is required to actuate the pump. Infusion devices having such functionality are fully described, for example, in copending application Serial Number 12/147,314 filed June 26, 2008 for DISPOSABLE INFUSION DEVICE WITH PRIME INDICATOR. The pump for this or any of the subsequent embodiments may be any one of acceptable drug delivery pumps which may include, for example, a piston pump, a peristaltic pump, a screw pump, a membrane pump, a metering device, and a gas driven positive displacement pump.

The base **40** and reusable portion **60** are releasably fixed together by a latch assembly **120.** In accordance with this embodiment, the latch assembly **120** includes a male part including projections **122** carried by the reusable portion **60** that are snappingly received within slots **124** of the base **40.** A further snap-action latch **126** is provided at the distal end of the system **20** to complete the confinement of the reusable portion **60** on the base **40.**

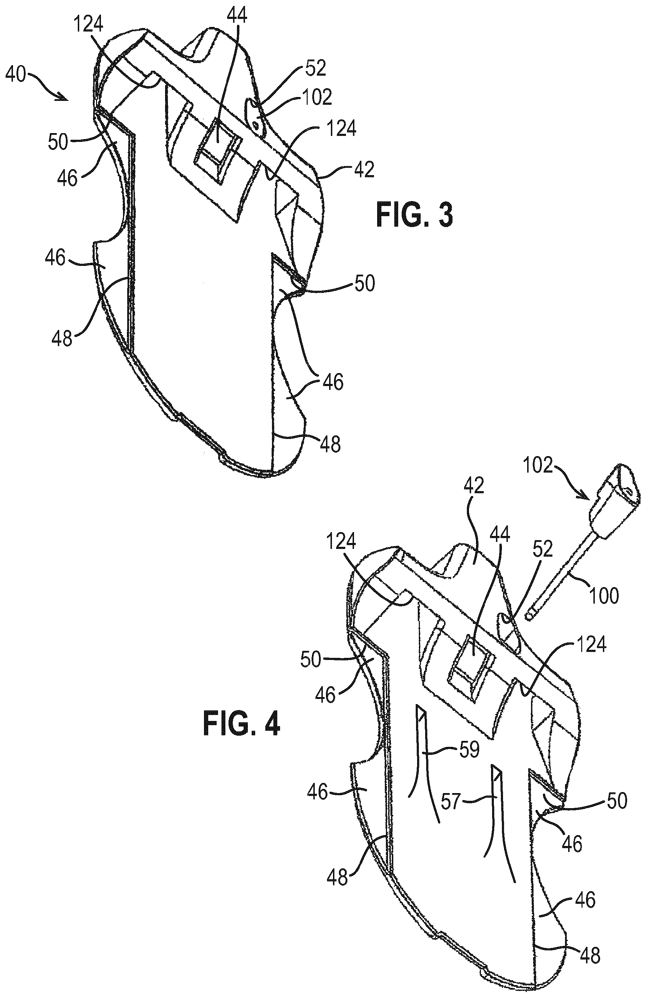

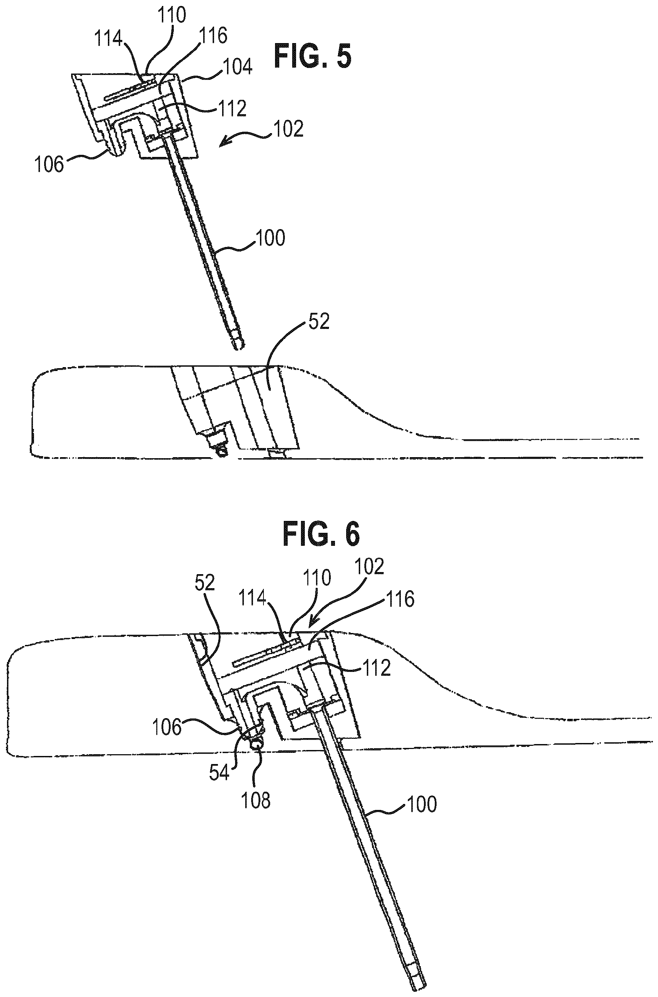

Alternatively, as shown in **FIG. 2B****,** the system **20'** comprises a reusable drug dispenser **60'** and a base portion **40',** which is releasably attached to the combined pump unit and reservoir **60'.** Similar to the other embodiments in this application, the base **40'** comprises an adhesive layer (not shown) configured to adhere to the skin of a wearer. The base also comprises a cannula well (similar to element **52** in **FIGS. 3** and **4****)** disposed in the base. Alternatively, the cannula well may be disposed in the reusable drug dispenser **60'.** To that end, the cannula set **102** (shown in **FIGS. 5** and **6****)** is used to drive cannula **100** through cannula exit port **101.**

**FIG. 2C** shows, in accordance with this embodiment, a cannula **106** that is provided as part of the base **40'.** In this embodiment, the base comprises a needle handle **105** covering the cannula port **101** on proximal (non-skin) side of the base. Needle handle **105** is attached a to detachable drive needle **107,** which is located on the distal side of the base **40'.** The drive needle **107** is in turn held within cannula **106** which is affixed to the distal side of base **40'.** Drive needle **107** is configured to introduce the cannula **106** into the skin.

In use, the wearer pushes the base **40'** against the wearer's skin, such that the needle **107** penetrates the skin. The cannula **106** is carried by the needle **107** through the tissue to beneath the skin. During this process and substantially simultaneously, the adhesive layer of the base **40'** will make contact with and adhere to the skin. **FIG. 2D** illustrates the assembly after the detachable drive needle is removed. This leaves the base **40'** attached to the skin **S,** and the cannula **106** extending through tissue beneath the skin.

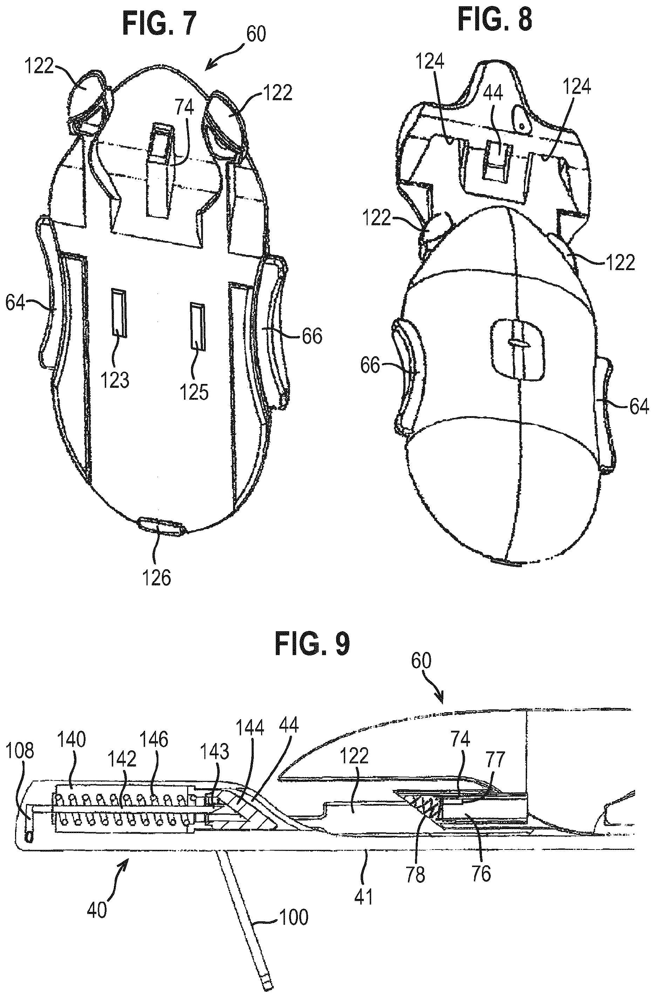

The perspective views of **FIG. 3** show the base **40** in greater detail. Here it may be seen that the base **40** includes a head portion **42.** The head portion **42** includes the slots **124** that snappingly receive the projections **122** (as shown in **FIGS. 7** and **8****)** of the reusable portion **60** when the base **40** and reusable portion **60** are joined together. The head portion also includes an opening **44** into which a coupling part of the reusable portion **60** is received to establish the antiseptic connection between the base **40** and the reusable portion **60.**

The base **40** further includes relieved surfaces **46** that form resulting shoulders **48** and **50.** The shoulders **48** and surfaces **46** form guides to guide the reusable portion **60** into proper alignment with the base **40** as they are joined together. The shoulders **50** provide a stop which is engaged when the base **40** and reusable portion **60** are finally snapped together. The opening **44** may also be formed in its proximal portion as a channel that mates with coupling projection **74** as shown in **FIGS. 7** and **9** to guide the aligned base and reusable portion into final and precise alignment for accurate attachment. Additionally or alternatively, grooves **57,59** in **FIG. 4** may mate with projections **123,125** shown in **FIG. 7** to help guide the two segments together in proper alignment.

As best seen in **FIG. 4****,** the base **40** further includes a well **52** that is arranged to receive a cannula set **102** that includes the cannula **100.** When the cannula set **102** is deployed, the cannula **100** is resultingly connected to an inlet within the head portion **42** and accessible through the opening **44** by the reusable portion.

The cannula set **102** and details of its deployment will now be described with particular reference to **FIGS.** 5 and **6****.** The cannula set **102** generally includes the cannula **100** and a cannula carrier **104.** The cannula carrier is dimensioned to fit accurately in the cannula well **52** of the head portion **42** of the base **40 (****FIG. 4****).** The carrier **104** includes a receiving pike **106** which is received by a correspondingly shaped feature **54** of the well **52.** The feature **54** is in fluid communication with a conduit **108** through which the drug, such as insulin, is caused to flow by the pump. The drug hence flows through the conduit **108,** through the feature pike **106,** and to the cannula **100** for delivery.

The carrier further includes a port **110** through which a deployment needle (not shown) may be inserted. Prior to cannula deployment, the deployment needle extends through the port **110**, through a passage **112,** and through the cannula **100.** The use of a deployment needle to subcutaneously place a cannula is described in greater detail in application 12/147,295 titled DISPOSABLE INFUSION DEVICE WITH AUTOMATICALLY RELEASABLE CANNULA DRIVER. The cannula set **102** is thus carried on the deployment needle. When the cannula set is deployed, the needle is retracted leaving the cannula set deployed as shown in **FIG. 6****.**

To preclude direct access to the cannula **100** through the port **110** after deployment needle removal, the cannula set further includes a port cover **114.** The port cover is preferably formed of resilient material and is arranged to spring over and block the port responsive to the drive needle being withdrawn from the port. Such a port cover is fully described, for example in co-pending application Serial Number 12/147,306 filed June 26, 2008 for DISPOSABLE INFUSION DEVICE WITH CANNULA PORT COVER. The port cover **114** together with a top **116** of the carrier **104** form a top sealing member of the carrier **104.**

**FIG. 7** shows the bottom view of the reusable portion **60.** Here it may be seen that the reusable portion **60** includes a coupling projection **74** that is arranged to be received by the opening **44** of the base **40** when the base **40** and reusable portion are attached. It may also be seen that the reusable portion **60** includes the latch **126** at its distal end to complete confinement of the reusable portion **60** on the base **40.**

**FIG. 8** shows the reusable portion **60** being attached to the base **40.** The projections **122** are aligned with and ready to be captured by the slots **124.** When the reusable portion **60** reaches its final position on the base **40,** it will cover essentially all of the base including the head portion **42 (****FIGS. 3** and **4****)** as shown in **FIG. 1****.**

**FIGS. 9-11** show the establishment of the fluid coupling between the base **40** and the reusable portion **60** as the reusable portion is brought into engagement with the base. **FIG. 9** shows the base **40** and reusable portion **60** prior to engagement.

Here the base **40** may be seen to include an inlet chamber **140.** Extending through the inlet chamber **140** is a needle **142** that forms an inlet to the base **40.** The needle **142** has a sharpened distal tip **143.** The distal end of the inlet chamber **140** is sealed with a self sealing, penetrable, barrier or septum **144.** A spring **146** urges the septum **144** in the distal direction. The reusable portion **60,** in turn, includes a conduit **76** within the coupling **74.** The coupling is sealed with a self sealing, penetrable, barrier or septum **78.** Immediately adjacent the septum **78** is a one-way valve **77** to vent the conduit **76.** This permits the drug, such as insulin, to be primed within the conduit so as to be in contact with the septum to eliminate air bubbles which might otherwise form.

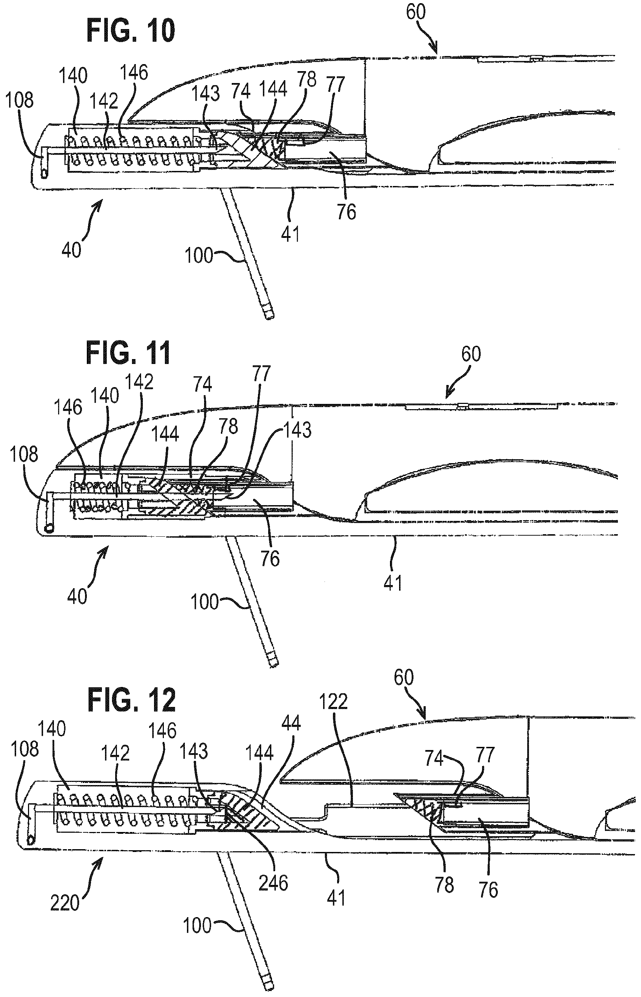

**FIG. 10** shows the base **40** and reusable portion **60** just as they engage. Here, it can be seen that the coupling **74** has entered the opening **44** and that the barriers **144** and **78** have engaged each other. When the reusable portion **60** reaches its final position on the base **40** as shown in **FIG. 11****,** the tip **143** of the inlet needle **142** has pierced through the septum **144** and the septum **78** to enter the conduit **76.** The spring **146** is also compressed. As a result, a sealed fluid connection is established from the conduit **76** in fluid communication with the pump, through the inlet needle **142,** through the conduit **108,** and to the cannula **100** for drug delivery.

When it is necessary to remove the reusable portion **60** from the base **40,** as the reusable portion **60** is pulled from the base **40,** the compressed spring **146** forces the septum **144** distally until it once again seals the inlet chamber **140** as shown in **FIG. 9****.** In addition to the inlet of the base **40** being sealed, the needle **142** is safely retracted back into the inlet chamber **140** to protect the wearer from being accidently pierced by the needle **146.** More specifically, the opening **44** to the inlet chamber **140** may be made small enough to eliminate the danger of even the smallest of fingers of children, for example, from gaining access to the inlet chamber **140** and pushing the septum **78** in toward and being pierced by the needle tip **143** during the handling of the base **140.**

**FIG. 12** shows another drug infusion system **220** The system **220** is essentially identical to the system **40,** previously described, and hence reference numerals for identical elements have been repeated herein and the description thereof is incorporated herein by reference. In addition to all of the elements of the system **40,** the system **220** of **FIG. 12** also includes an antiseptic wiper **246** within the inlet chamber **140** between the needle tip **143** and the septum **144.** The antiseptic wiper **246** may be compressible foam or cotton or the like. It is provided for wiping the needle **142** whenever it is caused to pierce the septum **144** or be withdrawn through the septum **144** as when the base **40** and reusable dispenser **60** are joined or separated. The wiper is preferably formed of a substance that will not plug or clog the needle and that will not constitute an irritant to the wearer should trace amounts thereof be injected with the delivered drug.

An alternative (not shown) to the small wiper **246** illustrated would be a larger block of compressible foam or cotton impregnated with antiseptic solution, the cotton or foam contained with the bore **140** and located so that it would extend slightly back from the tip of the needle **142** to pipe most of the needle except the tip with antiseptic solution whenever the septum **144** is forced out beyond the tip of the needle **142** by spring **146.**

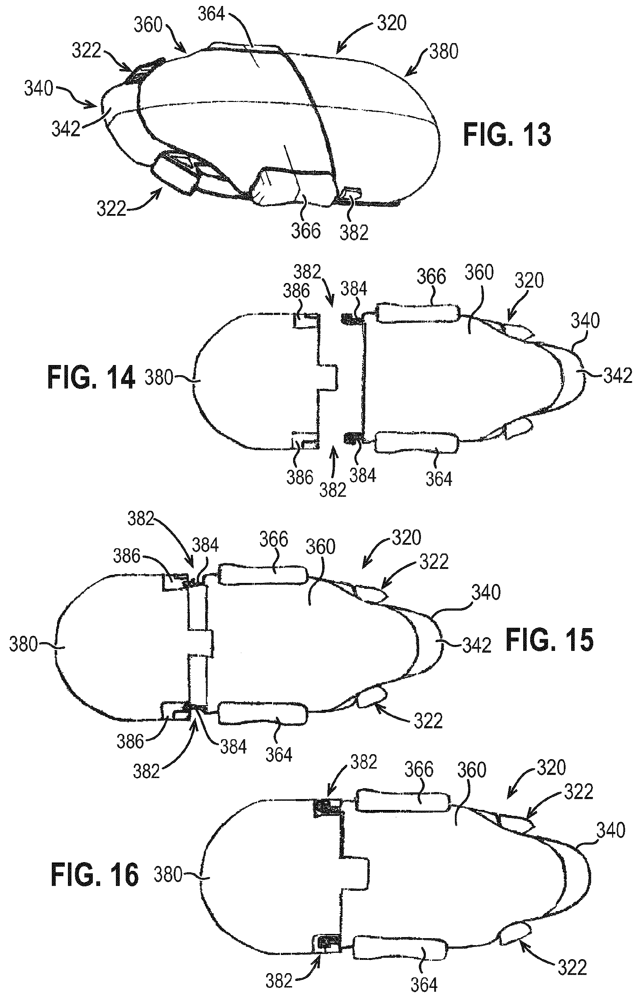

**FIG. 13** shows a further embodiment. Here, the drug infusion system **320** includes three primary components or portions; a base **340,** a reusable pump unit **360,** and a replaceable reservoir unit **380.**

The base **340** may be similar to the base **40,** previously described. To that end, it may also be arranged to receive a cannula set in its head portion **342** to establish fluid communication with the pump of the pump unit **360** in a manner as previously described.

The pump unit **360** is maintained on the base **340** by way of snap action latches **322** of the type previously described. The pump unit **360** includes actuator buttons **364** and **366** which, as previously described with respect to previous embodiments, are preferably arranged to cause drug delivery upon the concurrent depression of the actuator buttons **364** and **366.**

The reservoir unit **380** is maintained on the base **340** by way of side snap action latches **382.** The reservoir unit **380** is preferably prefilled prior to deployment in the system **320.** More particularly, the reservoir unit **380** may be provided as a stand alone item from a drug manufacturer under prescription and not require any special handling by the patient except for its deployment on the base **340** in engagement with the pump unit **360.** Alternatively, the reservoir unit may also be deployed on the reusable drug dispenser portion.

**FIG. 14** shows the pump unit **360** and reservoir unit **380** in alignment for engagement. The side snap action latches **382** each comprises a latch projection **384** carried by the pump unit **360** and a receiving slot **386** formed in the reservoir unit **380.** As may be seen in **FIG. 15****,** to join the pump unit **360** with the reservoir unit **380** on the base **340,** it is only necessary to advance the latch projections **384** into the receiving slots **386.** Once this is accomplished, the system is fully engaged as shown in **FIG. 16****.**

Antiseptic coupling of the base **340** and pump unit **360** and of the reservoir unit **380** and pump unit **360** may each be accomplished by employing dual septa and penetrating inlet needles as previously described. However, a vent need not be required for the antiseptic coupling of the reservoir unit **380** and the pump unit **360** because the reservoir unit may be manufactured to have the liquid drug, such as insulin, already immediately adjacent its sealing septum to prevent air bubble formation.

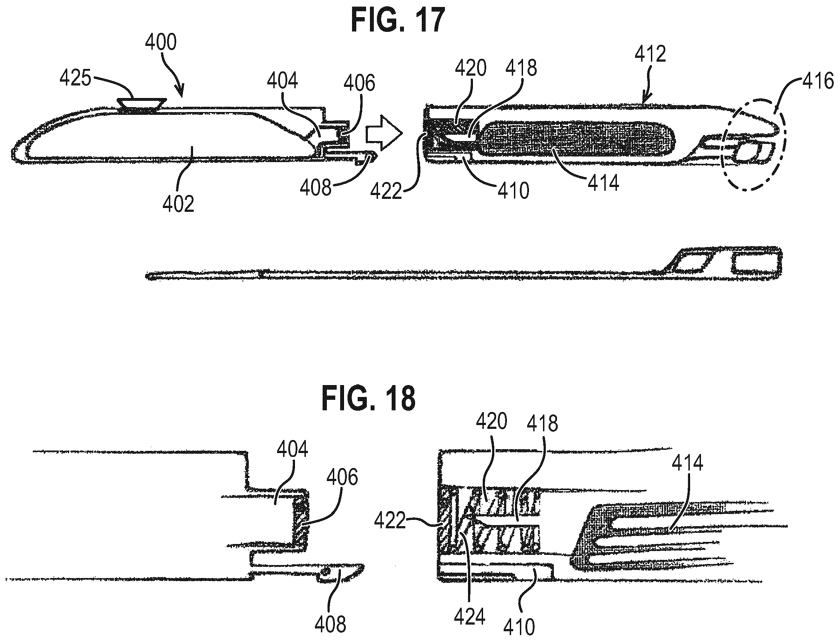

A more detailed description of the attachment mechanism of the reservoir portion and the pump portion may be seen in **FIGS. 17** and **18****.** The reservoir portion **400** contains a collapsible reservoir **402** which is fluidly connected to an outlet bore **404.** The collapsible reservoir may be characterized as a reservoir having a volume that decreases as fluid is expelled therefrom. Such a reservoir may be formed, for example, from flexible materials, or from rigid materials, having an internal moving component that decreases the volume within. The outlet bore is sealed with a piercable septum **406.** The reservoir portion is further provided with a male snap projection **408** which is configured to releasably mate with a female receptacle **410** in the pump portion **412.**

The pump portion **412** contains a pump **414** shown here in representative form. As stated earlier, any suitable pump may be employed. The pump portion contains on its distal end **416** all the mechanisms necessary to mate and form a detachable fluid connection with the base as described in detail above. In addition it contains a piercing needle **418** in an inlet bore **420.** Located at the end of the bore is a sealing septum **422.** A biasing mechanism, such as spring **424** urges the septum outward within the inlet bore **420.** The septum is movable with the bore, and when the two septa **406,422** are urged against each other, the inlet bore septa slides back over the piercing needle **418** which simultaneously pierces the reservoir septum and forms a fluid connection between the reservoir and the pump.

As with the previous connection between the reusable portion and the base, an antiseptic member may be provided within the bore and surrounding the needle **418** to wipe the needle between connections. It is also to be noted that the previous description of the connection between the reservoir portion and the pump portion illustrated a side releasable snap configuration similar to the snap attachment between the reusable portion and the base, and in the embodiment shown in **FIGS. 17** and **18****,** a bottom releasable attachment is illustrated. Likewise a top releasable attachment can easily be configured similar to the bottom releasable attachment shown.

The reservoir portion may be provided with a collapsible reservoir and prefilled by the manufacturer, in which case no priming mechanism is needed. If it is filled by the user soon before use, as is described in detail in the applications referenced herein, a simple mechanism of venting would be required. A vent comprised of a hydrophobic vent covered with a one way valve as described for the reusable unit and located on the outlet bore near the septum, in combination with a method of applying pressure to the reservoir such as a pressure button **425** would suffice.

In another embodiment, the reservoir may be a commercially available cartridge, such as an insulin cartridge. Such cartridges may be specially manufactured to fit the device, or may be of the type that is presently commercially available for syringe-pen injection units.

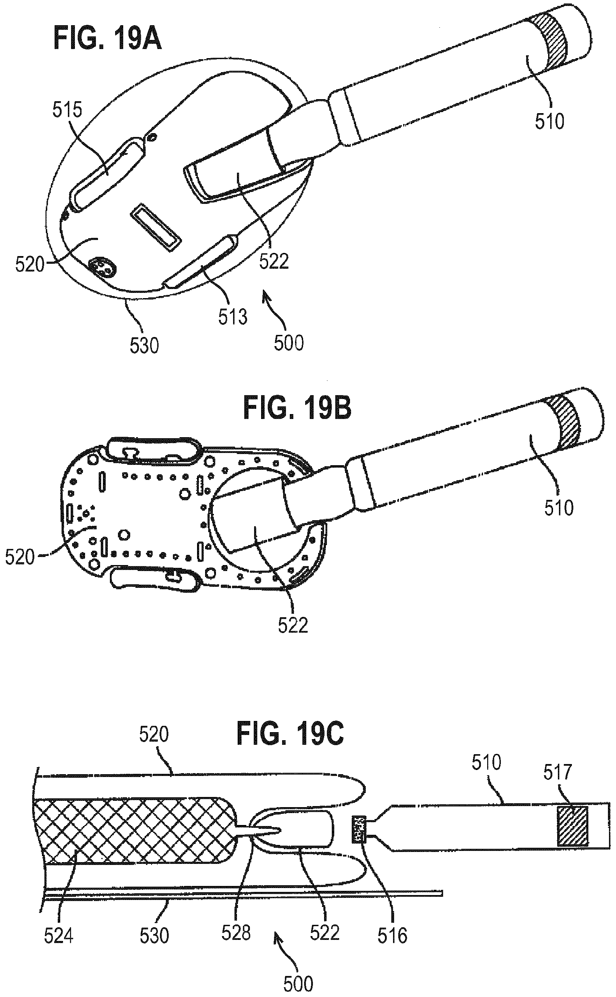

One possible embodiment using a commercially available cartridge (either pre-loaded or user loaded) is shown in **FIGS. 19A** through **19C****.** In this embodiment, as in the previous one, the pump unit of the reusable drug dispenser is configured to receive the reservoir. In accordance with this embodiment, the reservoir may be a commercially available cartridge, such as a glass syringe-pen cartridge (e.g., Humalog^{®} or Humulin^{®} sold by Lilly). As shown in **FIGS. 19A** and **19B****,** the device **500** comprises a cartridge reservoir **510,** a reusable drug dispenser in the form of a pump unit **520** and an adhesive base layer **530.** The cartridge reservoir **510** is received and maintained on the pump unit **520** by way of a receiving unit **522** that is in fluid communication with the cannula to the patient (not shown). As seen in **FIG. 19C****,** the cartridge reservoir **510** typically contains a septum **516** at the distal end of the cartridge and a plunger **517** at the proximal end. The receiving unit **522** comprises a hollow penetrating inlet needle **528** configured to pierce the septum **516** of the cartridge. Receiving unit **522** is configured to extend beyond the tip of needle **528** such that the needle **528** is not exposed outside the device. This precludes a user (also defined as a wearer) from being accidentally pricked by the needle **528.**

Needle **528** is in fluid communication with a pumping mechanism **524,** which can be any of the pumping mechanisms previously described. The pumping mechanism (also called a pump) in turn is in fluid communication with a cannula (not shown).

In use, the user inserts reservoir **510** (if the pump unit is not already pre-loaded) into the receiving unit **522** with sufficient force to pierce the septum **516.** Alternatively, the septum **516** may be pierced by the needle **528** by user activation after the user inserts it into the receiving unit. In some embodiments, it may be desirable that after inserting and securing a first reservoir, the pump unit is rendered unable to receive any subsequent reservoirs. This would make the reusable pump unit usable for the contents of just one reservoir. Once the needle **528** has pierced through the septum **516,** the fluid contained within the reservoir is drawn via the needle **528,** through the pumping mechanism **524** and out through the cannula (not shown) into the patient. The pumping mechanism **524** may be actuated by the concurrent depression of actuator buttons **513** and **515 (****FIG. 19A****)** contained on the body of the device **500,** as for example, on pump unit **520.** When the user actuates the pumping mechanism **524,** it draws fluid out of the reservoir **510,** and delivers it into the cannula.

In this and subsequent embodiments, the pre-filled cartridge **510** may comprise a plunger element **517** as best seen in **FIG. 19C****.** As the pumping mechanism **524** operates to draw liquid from the reservoir **510,** the suction created serves to pull the plunger **517** towards the septum **516.** The position of the plunger **517** may provide the user with a visual indication as to how much insulin remains within the reservoir. For example, some presently available insulin cartridges are equipped with visual volume indicators. Such markings may be calibrated to the amount of liquid left in the reservoir.

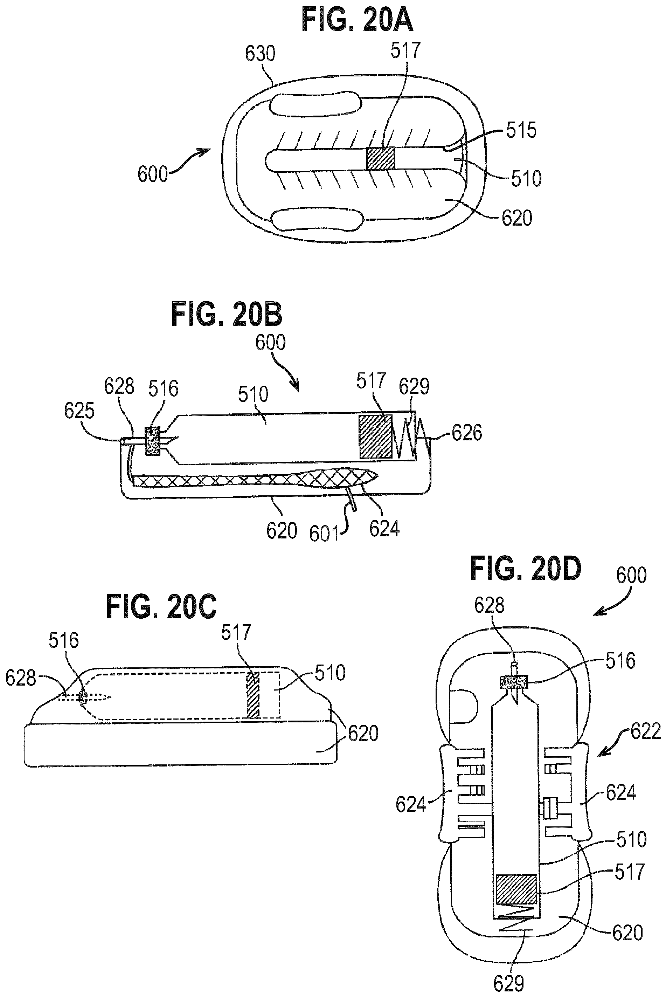

An alternative embodiment wherein a currently commercially available cartridge is employed as a reservoir is shown in **FIGS. 20A** through **20C****.** In this embodiment, a receiving unit is oriented to be in communication with a pre-filled cartridge that sits on top of the drug delivery device. Further, in this embodiment, the top of the device is configured to receive the pre-filled cartridge, for example, through a cavity that corresponds to the shape of the pre-filled cartridge.

As shown in **FIG. 20A****,** the drug delivery device **600** comprises a reservoir unit **510, a** reusable drug dispenser in the form of a pump unit **620** and a base adhesive layer **630.** The reservoir unit **510** may be a pre-filled cartridge. Pump unit **620** may comprise an elongated cavity that corresponds to the shape of the pre-filled cartridge reservoir **510.** For example, if the cartridge reservoir has a cylindrical configuration, the cavity preferably has a corresponding tubular configuration. The cavity **515** is configured to receive the reservoir **510** such that the reservoir is oriented parallel to the device.

As previously described in connection with **FIG. 19C** and in accordance with this embodiment, the cartridge **510** has a septum **516** at a first end and a plunger **517** disposed at a second, opposite, end. As seen in **FIG. 20B****,** the cavity of the pump unit comprises first receiving unit **625** configured to receive the first end of the cartridge **510** and a second receiving end **626** that is configured to receive the second end of the cartridge **510.** The receiving end **626** may further comprise a spring **629** that is configured to stabilize the reservoir within the cavity and/or to push the plunger **517** towards the septum **516.** In pushing the plunger towards the septum, the spring may provide additional driving force to compliment the suction offered by the pump to expel the liquid from the reservoir into the needle **628** or to help the created suction overcome an initial resistance against movement of the plunger.

The penetrating inlet needle **628** is disposed in the receiving unit **625.** As in previous embodiments, needle **628** is configured to pierce the septum **516** of the reservoir **510.** In this embodiment, receiving unit **625** might be just the needle **628** anchored into the rest of the device. Alternatively, it may comprise the needle **628** and a suitable covering for the needle; for example, a tube, a hood or other such suitable sheath, to ensure that a user does not come into contact with the needle. Optionally, the cavity may be covered to provide a tubular opening into which the pre-filled glass cartridge may be located. Such an embodiment is contemplated in **FIG. 20C****.** In this embodiment, an optional window (not shown) may be provided for visual indication of the amount of fluid left in the device.

As seen in **FIG. 20D****,** the pumping unit **620** includes a pumping mechanism **622** that includes a pair of actuating buttons **624.** The actuating buttons **624** are disposed within the pump unit **620** to accommodate the cavity that will house the reservoir **510.** More specifically, the pumping mechanism **622** (also referred to as a pump) may be configured such that one actuating button resides on one side of the cavity, and the other actuating button resides on the other side of the cavity. The actuating buttons **624** may carry attendant pump features distributed equally on opposite sides of the cavity.

In use, the cartridge reservoir **510** is placed into the cavity **515** of the pumping unit **620** such that the septum **516** contacts and is penetrated by the penetrating inlet needle **628.** The penetrating inlet needle **628** is in fluid communication with the pumping mechanism **622,** which can be any of the pumping mechanisms previously described. The pumping mechanism in turn is in fluid communication with cannula **601 (****FIG. 20B****).** The pumping mechanism is user actuated by, for example, the concurrent depression of the actuator buttons **624** on the body of the device **600,** for example on pump unit **620.** When the user actuates the actuator, the pumping mechanism **624** draws fluid out of the reservoir **510,** and delivers it to the cannula. In embodiments employing spring **629**,the spring may facilitate the drawing of fluid by the pump either by creating a continuous pressure throughout the course of use, or by helping overcome friction during the first actuation.

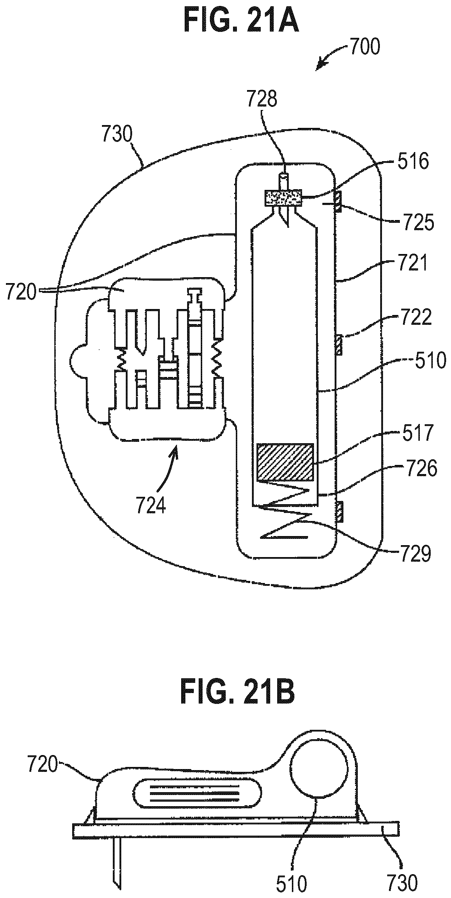

A further embodiment is shown in **FIGS. 21A** and **21B. FIGS. 21A** and **21B** show an embodiment in top perspective and end perspective views,
respectively. In this embodiment, a device **700** comprises a removable reservoir **510,** a reusable drug dispenser in the form of a pump unit **720,** and an adhesive base layer **730.** Pump unit **720** comprises a pumping mechanism **724** (also referred to as a pump) and an additional encasing unit **721.** Encasing unit **721** is configured to house the reservoir **510.** The encasing unit **721** is oriented to one side of the pumping mechanism **724.** This design lowers the vertical profile of the device by allowing the reservoir **510** to be housed beside the pumping mechanism **724.** However, if profile is not a concern, the encasing unit **721** may be placed in any orientation relative to the device, as for example, on top of the device, as seen for example in **FIG. 20C****,** where the reservoir is encased on top of the pump unit. The encasing unit **721** additionally comprises one or more securing mechanisms **722** which may take the form of locking tabs to secure the reservoir **510** within encasing unit **721.** Optionally, a window (not shown) may be provided on the encasing unit **721** to allow visualization of the plunger position and hence the amount of fluid left in the cartridge.

As in the previous embodiments, the pump unit comprises a receiving end **725** and a base-end **726.** The receiving end **725** comprises a penetrating inlet needle **728,** which is configured to penetrate the septum **516** of the reservoir. The base end **726** optionally comprises a spring **729** that is configured to push the plunger **517** of the device, thereby assisting with fluid entry into the needle **728.**

As in the previous embodiments, in use, a user places the reservoir **510** into the pump unit **720** such that the septum **516** contacts and is pierced by the needle **728.** The needle **728** is in fluid communication with the pumping mechanism **724** which draws fluid out of the reservoir **510** and into the cannula (not shown). In embodiments employing spring **729,** the spring facilitates the drawing of fluid by the pump either by creating a continuous pressure throughout the course of use, or by helping overcome friction during the first actuation.

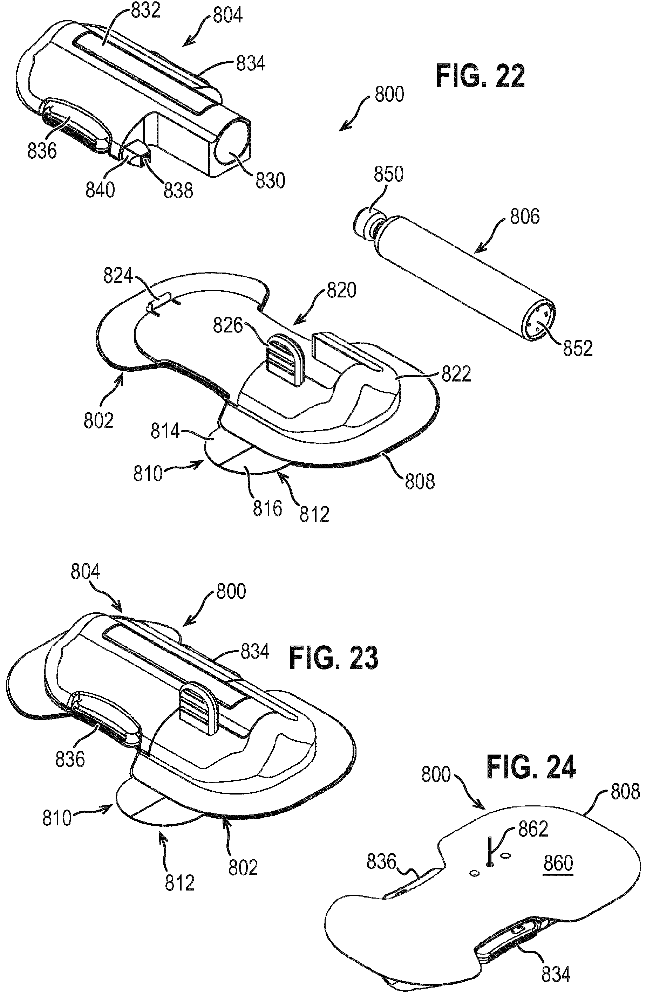

Referring now to **FIG. 22****,** it shows another assembly **800** in exploded and perspective view. As in prior embodiments, the assembly is a three component assembly including a base **802,** a pump assembly **804,** and a cartridge reservoir **806.**

The base **802** includes a flexible web **808** which has an adhesive thereon to permit the base to be adhered to the skin of a patient. Covering the adhesive are two tabbed covers **810** and **812** including tabs **814** and **816** respectively. The tabs allow the covers to be readily peeled off to expose the adhesive just prior to deployment of the base against the patient's skin.

The base **802** further includes a receiving structure **820** secured to the top surface of the web **808.** The receiving structure is arranged to detachably receive the pump unit **804** therein and includes a housing **822** arranged to receive and confine the forward end of the pump unit **804.** The receiving structure further includes a latch **824** that releasably locks the pump unit **804** onto the base **802.**

As will be seen subsequently, the base **808** includes a built-in cannula that extends from the adhesive side of the base **808.** To facilitate deployment of the cannula as the assembly is adhered to the patient's skin, the base also includes an insertion needle of the type known in the art that extends through the cannula and carries it to a deployed position. As will also be seen subsequently, after the assembly is deployed, the insertion needle may be pulled out of the cannula and the housing. To that end, a handle **826** connected to the insertion needle is provided. After deployment of the assembly **800,** the handled **826** may be grasped and pulled to remove the insertion needle.

The pump unit **804** includes an elongated cavity **830** for receiving the cartridge reservoir **806.** The cavity, in accordance with this embodiment, has a tubular shape to correspond to the generally cylindrical shape of the cartridge reservoir **806** as may be noted in the drawing. The pump unit **804** may include a window **832,** through which the amount of fluid left in the cartridge reservoir may be observed.

In accordance with prior embodiments, the pump unit **804** may contain any one of the pump mechanisms previously described herein. Actuation of the pump unit **804** may be achieved through a pair of actuating buttons **834** and **836.** Preferably, the pump unit **804** is arranged so that concurrent depression of the actuating buttons **834** and **836** is required to actuate the pump unit **804.**

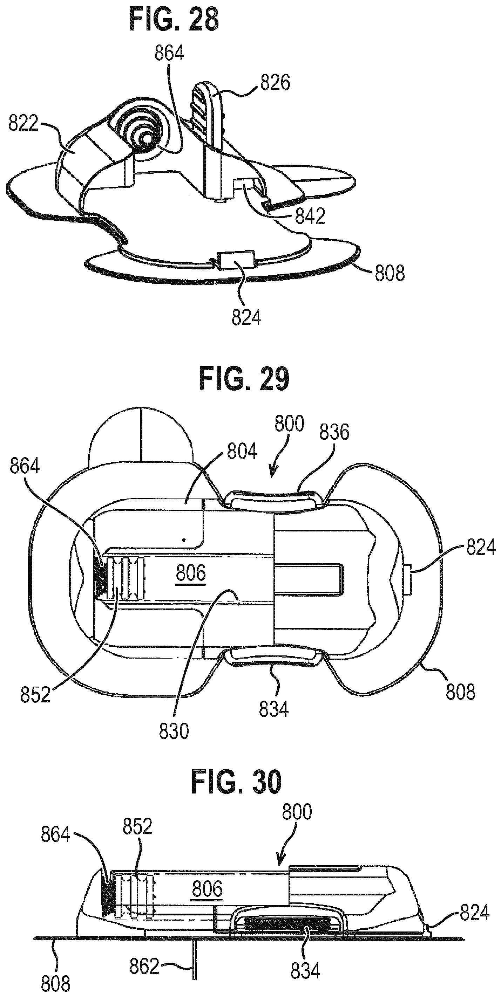

In accordance with this embodiment, when the pump unit **804** is actuated, a bolus of the fluid carried in the cartridge reservoir **806** is dispensed out of on outlet port **838** of the pump unit **804.** The outlet **838** is defined by a fitting **840** that makes a fluid tight seal with a corresponding inlet **842 (****FIG. 28****)** of the housing **822.** The inlet **842** is in fluid communication with the cannula to cause the bolus of fluid to be delivered to the cannula.

The cartridge reservoir **806** may be of the type previously described. It includes a septum **850** at its distal end and a plunger **852** at its proximal end. The plunger, as in prior embodiments, is arranged to translate along the length of the cartridge reservoir as fluid is removed therefrom. The position of the plunger **852** may be seen through the window **832** to provide the wearer with an indication as to how much fluid is remaining in the cartridge reservoir **806.**

**FIG. 23** shows the components of the assembly **800** assembled into an infusion device. The cartridge reservoir (not shown in **FIG. 23****)** has been loaded into the pump unit **804.** The pump unit **804** in turn has been releasably secured to the base **802.** The assembly, after the tabbed covers **810** and **812** are removed, will be ready to be deployed on a patient.

**FIG. 24** shows the bottom of the device **800** after the tabbed covers are removed to expose the adhesive surface **860** of the flexible web **808.** As may also be seen in **FIG. 24****,** the cannula **862** extends through the flexible web **808.** As will be seen herein after, the cannula **862** is protected by a protective cover that may be removed just prior to device deployment.

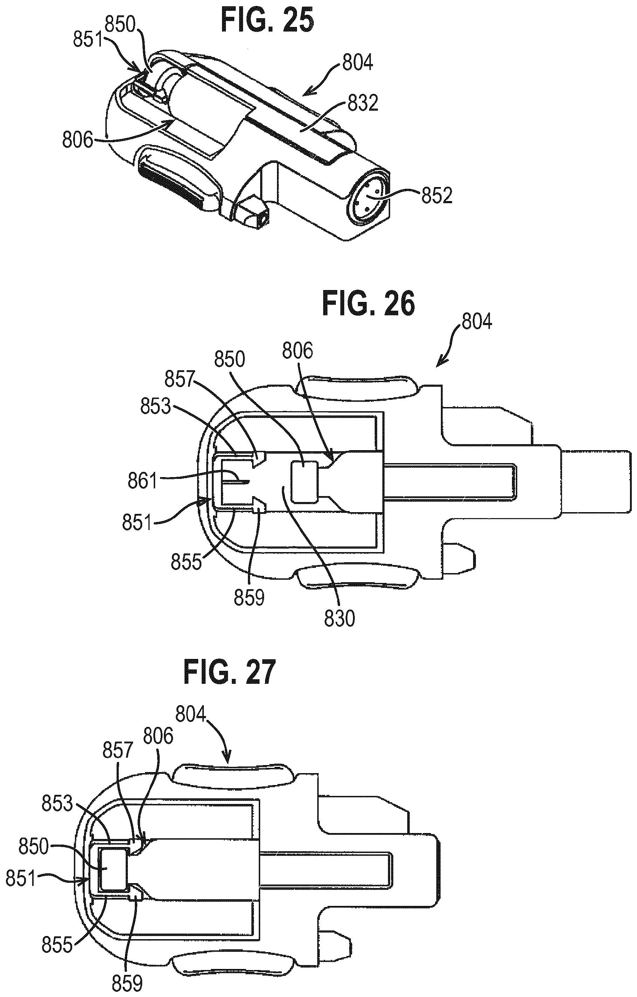

Referring now to **FIG. 25****,** it is a perspective view, with portions cut away, illustrating a releasable lock of the cartridge reservoir **806** within the pump unit **804.** Here the cartridge reservoir has been fully loaded into the pump assembly **804.** A latch assembly **851** firmly holds the cartridge reservoir **806** in place while also permitting the cartridge reservoir **806** to be removed from the pump unit **804** if necessary or desired.

**FIG. 26** illustrates the latch assembly **851** in greater detail. Here it may be seen that the latch assembly **851** is substantially U-shaped having extensions **853** and **855.** The extensions **853** and **855** extend into the cavity **830** of the pump unit **804** and terminate in latching ends **857** and **859** respectively. The extensions **853** and **855** are of sufficient length to fully encompass the septum **850** of the cartridge reservoir **852** when the cartridge reservoir **852** is fully loaded into the pump unit **804.**

**FIG. 26** also shows a needle **861.** The needle **861** serves to penetrate the septum **850** when the cartridge reservoir **852** is fully loaded into the pump unit **804.** This provides the fluid connection between the cartridge reservoir **806** and the pump mechanism (not shown). Since the needle **861** is deep within the cavity **830** of the pump unit **804,** protection against accidental contact with the needle is provided.

**FIG. 27** shows the cartridge reservoir **806** after being loaded into the pump unit **804** and being releasably locked therein by the latch assembly **851.** The septum **850** of the cartridge reservoir **806** is fully captured by the extensions **853** and **855** and their latching ends **857** and **859** respectively. The extension **853** and **855** are resilient for deflection to allow the septum **850** to enter past the latching ends **857** and **859.** This also allows the septum **850** to be withdrawn past the latching ends **857** and **859** when removal of the cartridge reservoir **806** from the pump unit **804** is necessary or desired.

**FIG. 28** is an end view, in perspective, of the base unit of the assembly of **FIG. 22****.** Here, it may be seen that the housing **822** of the base **802** includes a spring **864** arranged to engage the plug **852** of the cartridge reservoir **806 (****FIG. 22****).** Although a coiled spring is illustrated, it should be apparent to those skilled in the art that the spring may take different forms, such as for example, a leaf spring.

**FIGS. 29-34** illustrate the functioning of the spring **864.** As may be seen in **FIGS. 29 and 30****,** when the cartridge reservoir **806** is originally received within the cavity **830** of the pump unit **804,** the spring **864** contacts the plunger **852** of the cartridge reservoir **806.** The spring **864** becomes compacted to store energy and is now ready to assist the pulling of fluid from the cartridge reservoir **806** during the first actuation of the device **800** to overcome any friction that may otherwise preclude movement of the plug **852** within the cartridge reservoir **806.**

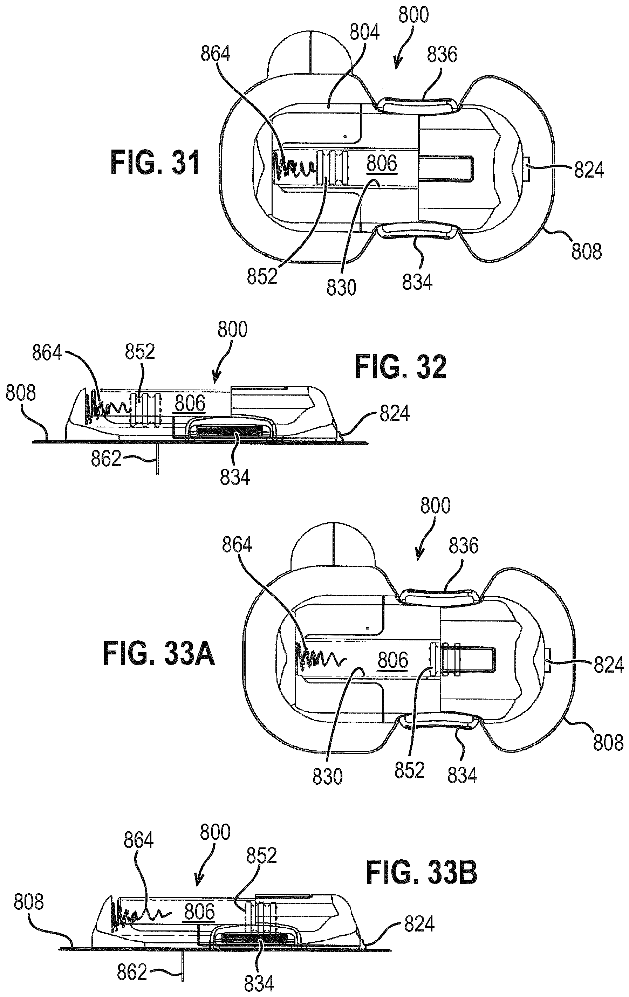

As may be seen in **FIGS. 31 and 32****,** the spring **864** is of sufficient length so that as fluid continues to be drawn from the cartridge reservoir **806,** it will assist in the movement of the plunger **852.** In some embodiments, the spring may only be required to free the plunger **852** for its initial movement. In that event, further spring function may be unnecessary permitting the spring to have a shorter axial free state length.

Eventually, as may be seen in **FIGS. 33A and 33B****,** the cartridge reservoir **806** will be almost empty and the plunger 852 will have moved far enough within the cartridge reservoir that the spring **864** will project into the cartridge reservoir **806** and will have lost contact with the plunger **852.**

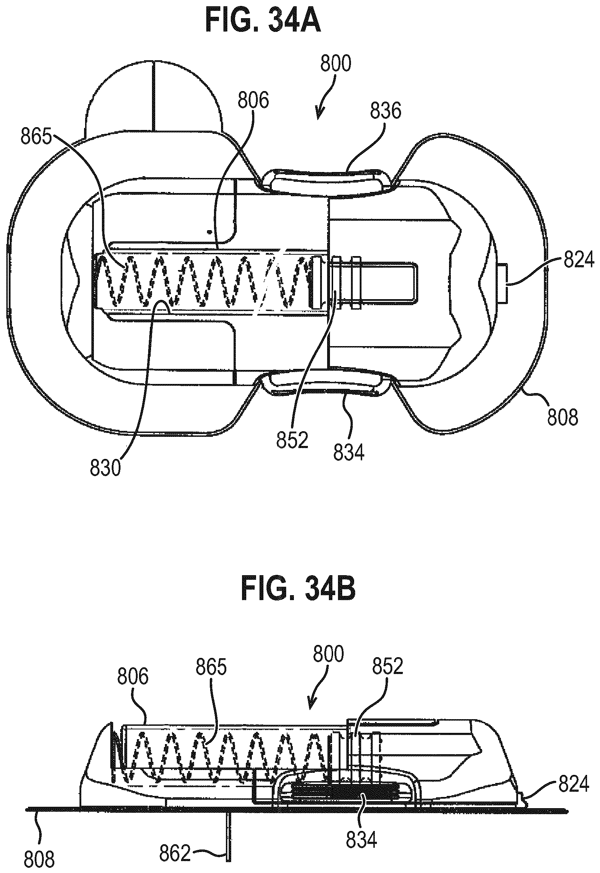

**FIGS. 34A and 34B** illustrate a further embodiment of the compression spring. Here, the compression spring **865** is of sufficient length to assist in the delivery of the fluid from the cartridge reservoir **806** until it is empty. As a result, the spring **865** remains in constant contact with the plunger **852** throughout its travel through the cartridge reservoir **806.**

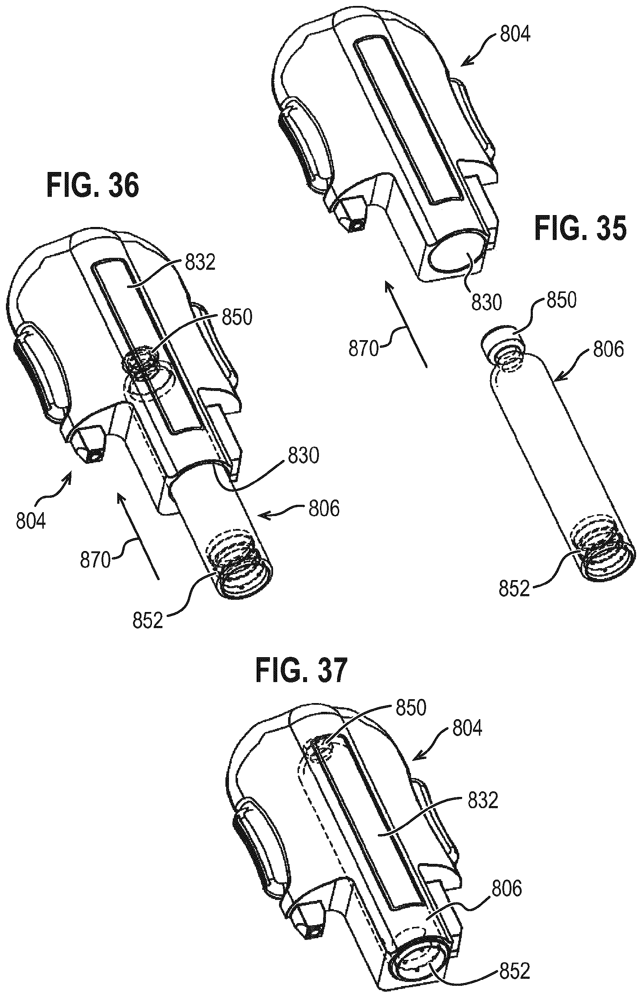

**FIGS. 35-39** show the sequence of steps to be performed to make the assembly **800** of **FIG. 22** ready for deployment. As may be seen in **FIG. 35****,** the cartridge reservoir **806** is moved relative to the pump unit **804** in the direction of arrow **870** to insert the cartridge reservoir **806,** septum **850** end first, into the cavity **830** of the pump assembly. As may be seen in **FIG. 36****,** as the cartridge reservoir **806** is moved in the direction of arrow **870,** the septum **850** end of the cartridge reservoir **806** may be viewed through the window **832** to monitor the cartridge reservoir **806** insertion process. When the cartridge reservoir **806** is fully inserted into the pump unit **804,** the partial assemblage will appear as shown in **FIG. 37****.**

Preferably, the pump unit **804** includes cartridge receiving structures of the type previously described herein including a septum piercing needle to pierce the septum **850** and connect the cartridge reservoir **806** to a pump mechanism. The plunger **852** of the cartridge reservoir **806** protrudes slightly from the proximal end of the pump unit **804** and is ready to contact a spring of the base as previously described.

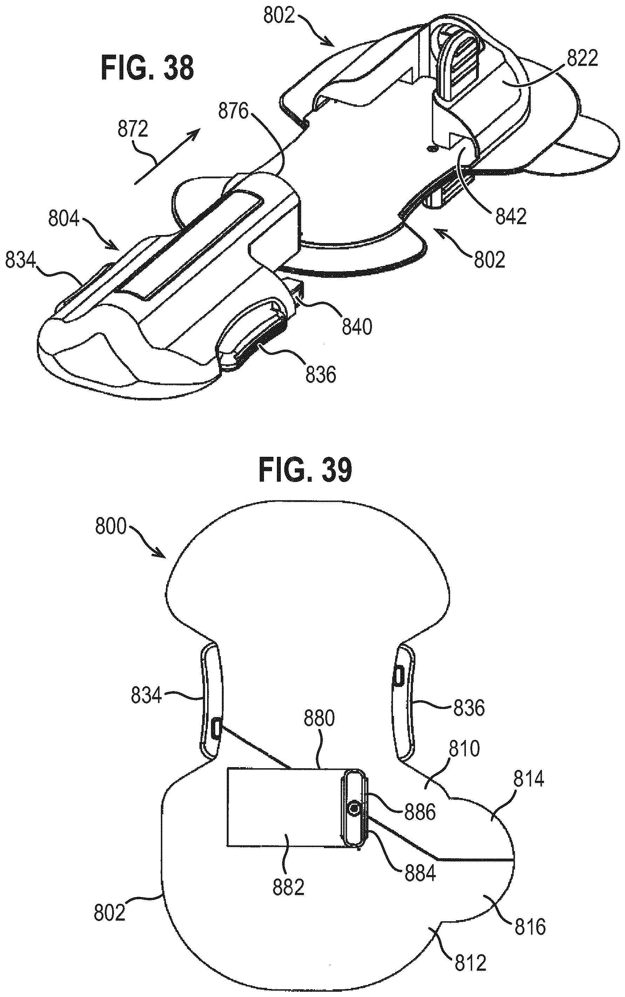

Next, the pump unit **804** is releasably joined with the base **802.** As seen in **FIG. 38****,** this is accomplished by sliding the pump unit **804** in the direction of arrow **872** until the proximal end **876** of the pump unit **804** is fully within the housing **822** of the base **802.** As the proximal end **876** of the pump unit **804** enters the housing **822,** the fitting **840** of the pump unit **804** will make a fluid tight seal with the inlet **842** of the housing **822** of the base **802.** This having been accomplished, the assembled assembly **800** will appear as previously shown in **FIG. 23****.**

Now, priming of the fluid delivery system and removal of the adhesive covering tabbed covers **810** and **812** are required. This may be accomplished as shown in **FIG. 39****,** which shows the bottom view of the device (i.e., the device **800** is turned over). A protective structure **880** protects the cannula from damage. More specifically, the protective structure **880** is substantially shaped and includes a horizontal portion **882** and a vertical portion **884** substantially transverse to the horizontal portion **882.** The vertical portion **884** includes a bore **886** having the cannula therein.

To prime the fluid delivery system, the device **800** is actuated by depressing the actuator buttons **834** and **836** enough times to cause fluid to appear out of bore **886.** When this occurs, it is known that the cannula and all of the fluid conduits from the cartridge reservoir to the distal end of the cannula are filled with fluid.

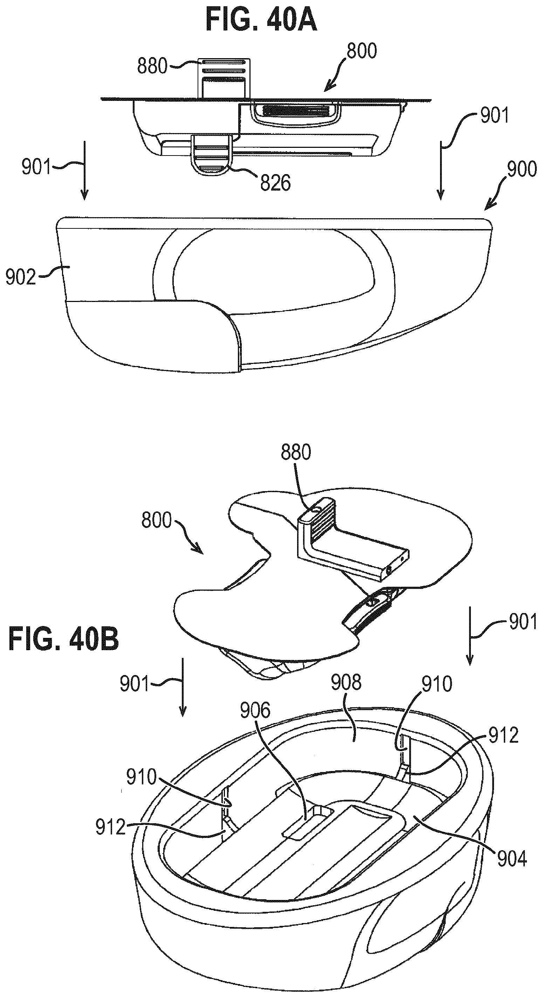

**FIGS. 40A and 40B** are exploded views of the infusion device **800** and an inserter **900** for deploying the device in accordance with further aspects. The inserter **900** includes a housing **902** dimensioned to reach the device **800.**

The device **800** may thus be placed into the inserter **900** in the direction of arrows **901.** The inserter housing **902** includes a moveable top **904**
that has an inner surface contour that matches the general surface contour of the device **800.** The top **904** has an opening **906** for receiving the insertion needle handle **826** that protrudes from the device **800.** The inserter housing **902** has a side wall **908** that includes guide channels **910.** The guide channels **910** slidingly receive guide extensions **912** that extend from the inserter top **904.** The guide channels **910** and guide extensions **912** serve to controllably guide the translation of the top **904,** and hence the device, during deployment of the device **800.** To that end, the top may be manually driven by the user or the top may be driven by a mechanical drive force as may be provided by the stored energy of a drive spring, for example.

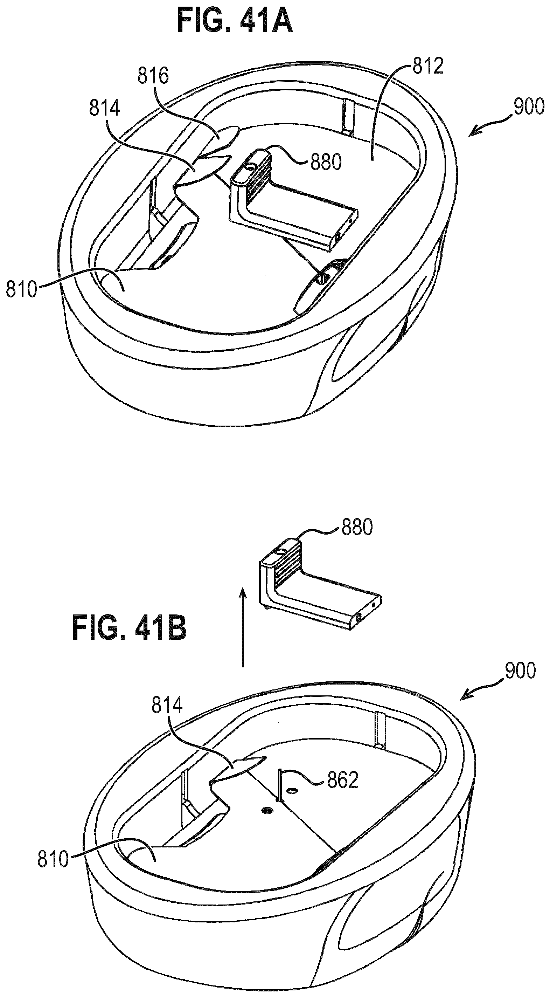

**FIGS. 41A and 41B** are perspective views of the infusion device **800** and inserter **900** after the infusion device has been loaded into the inserter. In the process of loading the device **800** into the inserter **900,** the tabs **814** and **816** of the tabbed adhesive protective covers **810** and **812** respectively are turned-up for ready removal. **FIG 41B** shows the cover **812** removed and cover **810** ready for removal. Also seen in **FIG. 41B** is the cannula protective structure **880** removed from the cannula. Once cover **810** is removed from the device **800,** the device **800** will be ready for deployment with the inserter.

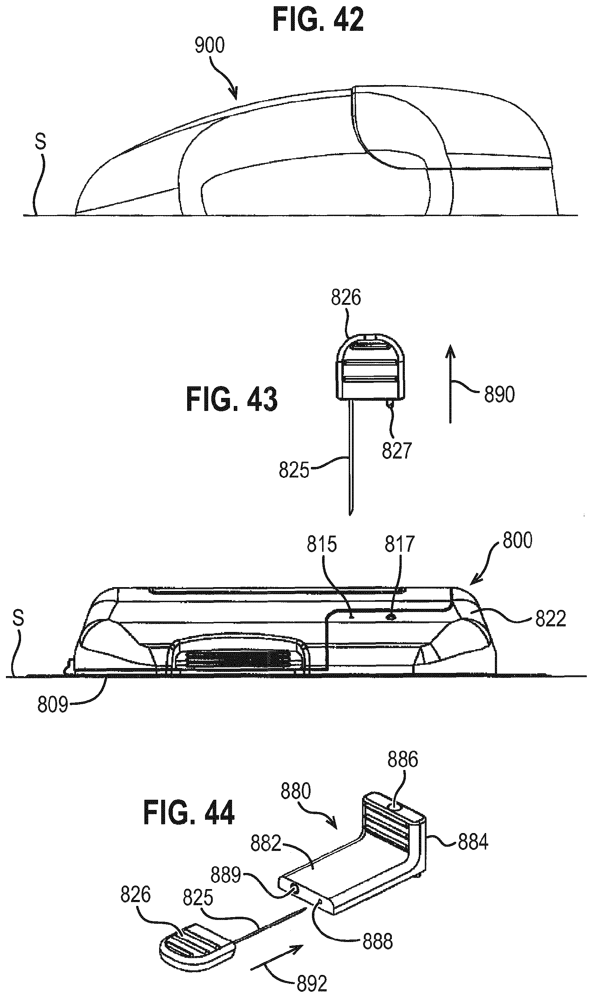

**FIG. 42** shows that the inserter has been placed against the skin **S** of the patient. Now, upon actuation of the inserter, either by manual force or released stored force, the entire device will be driven to the skin of the patient. This will cause the cannula and insertion needle to penetrate the patient's skin and the adhesive surface of the base of the device to contact and be adhered to the patient's skin.

**FIG. 43** shows the device **800** on the patient's skin **S** after the inserter has been removed. The base surface **809** of the device is adhered to the patient's skin. **FIG. 43** also illustrates the insertion needle **825** being pulled from the device **800** and more specifically the cannula (not shown) through a hole **815** in the base housing **822** of the device **800** in the direction of arrow **890.** The insertion needle is readily pulled by grasping the handle **826** of the insertion needle **825.** As may be further seen in **FIG. 43****,** the insertion needle handle **826** includes an alignment pin **827** that is being pulled from a corresponding hole **817** of the base housing **822.**

**FIG. 44** illustrates a preferred manner of storing the insertion needle **825** once it has been removed from the device. Here it may be seen that the needle **825** is stored in the protective structure **880** previously used for protecting the cannula of the device. To that end, the protective structure includes a hole **888** within the horizontal portion **882** for receiving the needle **825** as the needle is inserted therein in the direction of arrow **892.** The horizontal portion further includes a hole **889** for likewise receiving the alignment pin **827 (****FIG. 43****)** of the insertion needle handle **826.** The insertion needle is now ready for safe sharps disposal.

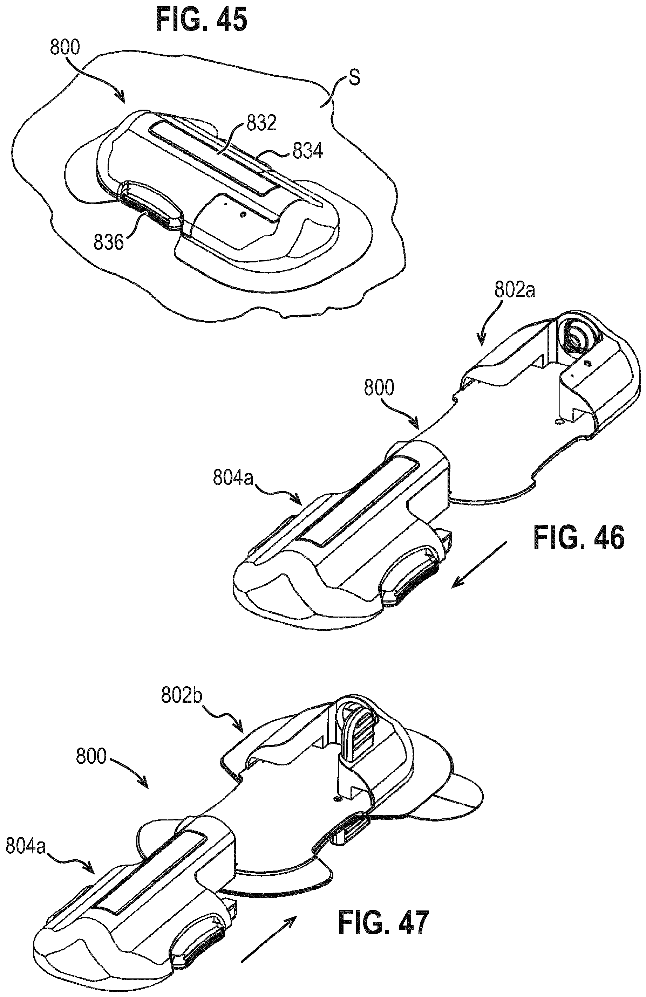

**FIG. 45** shows the device **800** fully deployed on the patient's skin S and ready to provide a first bolus of liquid medicament to the patient. While the device is in use, the amount of medicament remaining in the cartridge reservoir may be discerned by simply looking through the window **832** and noting the position of the cartridge plunger. As previously described, the device may be actuated to deliver each bolus of medicament preferably by the concurrent depression of the actuator buttons **834** and **836.**

**FIGS. 46 and 47** illustrate the convenience and flexibility afforded by this embodiment. Generally, a cannula should not be left in a subcutaneous position within a patient for more than about three days. Otherwise,
the infusion site could become infected. Hence,
it is possible that when it is time to remove a cannula, there may still be medicament remaining in the cartridge reservoir in use. In this event, the entire device may be removed from the patient but not discarded in its entirety. The pump unit may be reused. As a result, as seen in **FIG. 46****,** the pump unit in use **802a** may be removed from the old base **802a.** Then, as shown in **FIG. 47****,** the old pump unit **804a** may be releasably joined with a new base **802b.** Thereafter, the new assemblage of the new base **802b** and reused pump unit **804a** may be deployed as previously described.

Of course, should the cartridge reservoir of a pump unit become empty before it is time to remove the base and cannula, the spent pump unit may be simply removed from the base and replaced with a new pump unit having a new cartridge reservoir. Still further, it is possible to reuse a pump unit. Hence, if a base need not be removed but a cartridge reservoir becomes empty, the pump unit may simply be removed from the base, the spent cartridge reservoir may be removed from the pump unit, a new cartridge reservoir may be inserted into the pump unit, and the reused pump unit equipped with the new cartridge reservoir may be releasably joined with the base.

While particular embodiments of the present invention have been shown and described, modifications may be made. The scope of the invention is defined by the appended claims.

## Claims

1. A drug infusion system comprising:
a base (40) having a cannula well (52) arranged to receive a cannula (100) that conducts the drug to beneath a wearer's skin, the base (40) further including a base surface arranged to attach to the skin of the wearer, the base (40) including the cannula well (52) and arranged to dispose the cannula (100) to extend from the base surface to beneath the wearer's skin, the base (40) further including an inlet chamber (140) having a needle (142) with a sharpened distal tip (143) extending therethrough that forms an inlet arranged to receive the drug, a conduit (76) that conducts the drug from the inlet to the cannula well (52), and a first self sealing penetrable barrier (144) moveable with respect to the inlet;
a reusable drug dispenser (60) removably attachable to the base (40) and having a second self sealing penetrable barrier (78), a reservoir arranged to hold the drug, and a pump that pumps the drug to the second self sealing penetrable barrier, the first and second self sealing penetrable barriers (140, 78) being arranged to engage each other and be penetrated by the inlet of the base when the reusable dispenser is attached to the base (40) to form an antiseptic connection between the cannula well (52) and the reservoir; and
wherein the inlet of the base has a distal end that penetrates the first and second self sealing penetrable barriers (144, 78) when the reusable dispenser (60) is attached to the base (40), **characterized in that** the base (40) further includes a biasing element (146) that urges the first self sealing penetrable barrier (144) against the second self sealing penetrable barrier (78) when the reusable dispenser (60) is attached to the base (40).

2. The system of claim 1, further comprising a latch assembly (120) that releasably holds the reusable dispenser (60) on the base (40).

3. The system of claim 1, further comprising a cannula set (102) including a receiving pike (106) and the cannula (100), the receiving pike (106) being arranged to be received within the cannula well (52) in fluid communication with the conduit (76), whereby a fluid connection is formed from the cannula (100) through the conduit (76) to the reservoir.

4. The system of claim 1, wherein the base (40) includes a guide that guides the reusable dispenser (60) into attachment on the base.

5. The system of claim 1, wherein the base (40) is coextensive with the reusable dispenser (60) when the reusable dispenser (60) is attached to the base (40).

6. The system of claim 1, wherein the reusable dispenser (60) includes an inlet cavity adjacent the second self sealing penetrable barrier that receives the inlet of the base when the reusable dispenser (60) is attached to the base (40), the inlet cavity being arranged to receive the drug from the reservoir and provide the drug to the inlet of the base (40).

7. The system of claim 1, wherein the reusable dispenser (60) includes a pair of actuators operatively associated with the pump for causing the pump to pump the drug from the reservoir to the cannula (100) upon concurrent actuation of the actuators.

8. The system of claim 1, wherein the reservoir comprises a separate reservoir unit (380) that holds the drug to be delivered, the reservoir unit (380) being engageable with the reusable dispenser (360) on the base (340).

9. The system of claim 8, further comprising at least one latch (382) that maintains the reusable dispenser (360) and reservoir unit (380) in engagement, the at least one latch comprising a latching projection and a projection receiving slot.

10. The system of claim 1, further comprising a vent (77) immediately adjacent the second self sealing penetrable barrier (78).

## Patentansprüche

1. Medikamenteninfusionssystem, umfassend:
eine Basis (40), die eine Kanülenvertiefung (52) aufweist, die angeordnet ist, um eine Kanüle (100) aufzunehmen, die das Medikament bis unter die Haut eines Trägers leitet, wobei die Basis (40) ferner eine Basisoberfläche einschließt, die angeordnet ist, um an der Haut des Trägers angebracht zu werden, wobei die Basis (40) die Kanülenvertiefung (52) einschließt und angeordnet ist, um die Kanüle (100) einzurichten, um sich von der Basisoberfläche bis unter die Haut des Trägers zu erstrecken, wobei die Basis (40) ferner eine Einlasskammer (140), die eine Nadel (142) mit einer geschärften distalen Spitze (143) aufweist, die sich dahindurch erstreckt und einen Einlass ausbildet, der angeordnet ist, um das Medikament aufzunehmen, eine Leitung (76), die das Medikament von dem Einlass zu der Kanülenvertiefung (52) leitet, und eine erste selbstdichtende durchdringbare Sperre (144) einschließt, die in Bezug auf den Einlass bewegbar ist;
eine wiederverwendbare Medikamentenabgabeeinrichtung (60), die an der Basis (40) abnehmbar anbringbar ist und eine zweite selbstdichtende durchdringbare Sperre (78), ein Reservoir, das angeordnet ist, um das Medikament aufzunehmen, und eine Pumpe aufweist, die das Medikament zu der zweiten selbstdichtenden durchdringbaren Sperre pumpt, wobei die erste und die zweite selbstdichtende durchdringbare Sperre (140, 78) angeordnet sind, um miteinander in Eingriff zu kommen und durch den Einlass der Basis durchdrungen zu werden, wenn die wiederverwendbare Abgabeeinrichtung an der Basis (40) angebracht ist, um eine antiseptische Verbindung zwischen der Kanülenvertiefung (52) und dem Reservoir auszubilden; und
wobei der Einlass der Basis ein distales Ende aufweist, das die erste und die zweite selbstdichtende durchdringbare Sperre (144, 78) durchdringt, wenn die wiederverwendbare Abgabeeinrichtung (60) an der Basis (40) angebracht ist, **dadurch gekennzeichnet, dass** die Basis (40) ferner ein Vorspannelement (146) einschließt, das die erste selbstdichtende durchdringbare Sperre (144) gegen die zweite selbstdichtende durchdringbare Sperre (78) drückt, wenn die wiederverwendbare Abgabeeinrichtung (60) an der Basis (40) angebracht ist.

2. System nach Anspruch 1, ferner umfassend eine Verriegelungsanordnung (120), die die wiederverwendbare Abgabeeinrichtung (60) lösbar an der Basis (40) hält.

3. System nach Anspruch 1, ferner umfassend ein Kanülenset (102), das einen Aufnahmedorn (106) und die Kanüle (100) einschließt, wobei der Aufnahmedorn (106) angeordnet ist, um innerhalb der Kanülenvertiefung (52) in Fluidverbindung mit der Leitung (76) aufgenommen zu werden, wodurch eine Fluidverbindung von der Kanüle (100) durch die Leitung (76) zu dem Reservoir ausgebildet wird.

4. System nach Anspruch 1, wobei die Basis (40) eine Führung einschließt, die die wiederverwendbare Abgabeeinrichtung (60) zu einer Befestigung an der Basis führt.

5. System nach Anspruch 1, wobei die Basis (40) deckungsgleich mit der wiederverwendbaren Abgabeeinrichtung (60) ist, wenn die wiederverwendbare Abgabeeinrichtung (60) an der Basis (40) angebracht ist.

6. System nach Anspruch 1, wobei die wiederverwendbare Abgabeeinrichtung (60) einen Einlasshohlraum angrenzend an die zweite selbstdichtende durchdringbare Sperre einschließt, der den Einlass der Basis aufnimmt, wenn die wiederverwendbare Abgabeeinrichtung (60) an der Basis (40) angebracht ist, wobei der Einlasshohlraum angeordnet ist, um das Medikament aus dem Reservoir aufzunehmen und das Medikament dem Einlass der Basis (40) zuzuführen.

7. System nach Anspruch 1, wobei die wiederverwendbare Abgabeeinrichtung (60) ein Paar von Betätigungselementen umfasst, die der Pumpe funktionell zugeordnet sind, um zu bewirken, dass die Pumpe bei gleichzeitiger Betätigung der Betätigungselemente das Medikament aus dem Reservoir zu der Kanüle (100) pumpt.

8. System nach Anspruch 1, wobei das Reservoir eine separate Reservoireinheit (380) umfasst, die das auszugebende Medikament enthält, wobei die Reservoireinheit (380) mit der wiederverwendbaren Abgabeeinrichtung (360) auf der Basis (340) in Eingriff gebracht werden kann.

9. System nach Anspruch 8, ferner umfassend mindestens eine Verriegelung (382), die die wiederverwendbare Abgabeeinrichtung (360) und die Reservoireinheit (380) in Eingriff hält, die mindestens eine Verriegelung umfassend einen Verriegelungsvorsprung und einen Vorsprungaufnahmeschlitz.

10. System nach Anspruch 1, ferner umfassend eine Entlüftung (77) unmittelbar angrenzend an die zweite selbstdichtende durchdringbare Sperre (78).

## Revendications

1. Système de perfusion de médicament, comprenant :
une base (40) ayant un puits de canule (52) agencé pour recevoir une canule (100) qui conduit le médicament sous la peau d'un porteur, la base (40) comportant en outre une surface de base agencée pour se fixer à la peau du porteur, la base (40) comportant le puits de canule (52) et agencée pour disposer la canule (100) afin qu'elle s'étende de la surface de base jusqu'au-dessous de la peau du porteur, la base (40) comportant en outre une chambre d'entrée (140) ayant une aiguille (142) avec une pointe distale aiguisée (143) s'étendant à travers celle-ci qui forme une entrée agencée pour recevoir le médicament, un conduit (76) qui conduit le médicament de l'entrée au puits de canule (52), et une première barrière pénétrable auto-obturante (144) mobile par rapport à l'entrée ;
un distributeur de médicament réutilisable (60) pouvant être fixé de manière amovible à la base (40) et ayant une seconde barrière pénétrable auto-obturante (78), un réservoir agencé pour contenir le médicament, et une pompe qui pompe le médicament vers la seconde barrière pénétrable auto-obturante, les première et seconde barrières pénétrables auto-obturantes (140, 78) étant agencées pour venir en prise l'une avec l'autre et être pénétrées par l'entrée de la base lorsque le distributeur réutilisable est fixé à la base (40) pour former une connexion antiseptique entre le puits de canule (52) et le réservoir ; et
dans lequel l'entrée de la base a une extrémité distale qui pénètre dans les première et seconde barrières pénétrables auto-obturantes (144, 78) lorsque le distributeur réutilisable (60) est fixé à la base (40), **caractérisé en ce que** la base (40) comporte en outre un élément de sollicitation (146) qui pousse la première barrière pénétrable auto-obturante (144) contre la seconde barrière pénétrable auto-obturante (78) lorsque le distributeur réutilisable (60) est fixé à la base (40).

2. Système selon la revendication 1, comprenant en outre un ensemble loquet (120) qui maintient de manière amovible le distributeur réutilisable (60) sur la base (40).

3. Système selon la revendication 1, comprenant en outre un jeu de canules (102) comportant un pic de réception (106) et la canule (100), le brochet de réception (106) étant agencé pour être reçu à l'intérieur du puits de canule (52) en communication fluidique avec le conduit (76), moyennant quoi un raccordement fluidique est formé à partir de la canule (100) à travers le conduit (76) vers le réservoir.

4. Système selon la revendication 1, dans lequel la base (40) comporte un guide qui guide le distributeur réutilisable (60) jusqu'à ce qu'il soit fixé à la base.

5. Système selon la revendication 1, dans lequel la base (40) est coextensive au distributeur réutilisable (60) lorsque le distributeur réutilisable (60) est fixé à la base (40).

6. Système selon la revendication 1, dans lequel le distributeur réutilisable (60) comporte une cavité d'entrée adjacente à la seconde barrière pénétrable auto-obturante qui reçoit l'entrée de la base lorsque le distributeur réutilisable (60) est fixé à la base (40), la cavité d'entrée étant agencée pour recevoir le médicament du réservoir et fournir le médicament à l'entrée de la base (40).

7. Système selon la revendication 1, dans lequel le distributeur réutilisable (60) comporte une paire d'actionneurs associés de manière fonctionnelle à la pompe pour amener la pompe à pomper le médicament du réservoir vers la canule (100) lors de l'actionnement simultané des actionneurs.

8. Système selon la revendication 1, dans lequel le réservoir comprend une unité de réservoir séparée (380) qui contient le médicament à administrer, l'unité de réservoir (380) pouvant venir en prise avec le distributeur réutilisable (360) sur la base (340).

9. Système selon la revendication 8, comprenant en outre au moins un loquet (382) qui maintient le distributeur réutilisable (360) et l'unité de réservoir (380) en prise, l'au moins un loquet comprenant une saillie de verrouillage et une fente de réception de saillie.

10. Système selon la revendication 1, comprenant en outre un évent (77) immédiatement adjacent à la seconde barrière pénétrable auto-obturante (78).
